# EUROPEAN PATENT APPLICATION

(11) **EP 1 634 596 A1**
(43) Date of publication of application: **15.03.2006**
(21) Application number: 03732200.5
(22) Date of filing: 19.06.2003
(51) Int. Cl.: A61K 31/473, A61K 31/48, C07D 221/18, A61P 7/02

(54) **APORPHINE AND OXOAPORPHINE AND THE MEDICAL USE THEREOF**

(71) Applicant: Lotus Pharmaceutical Co., Ltd., Taipei 106 (TW)
(72) Inventor: SU, Mingjai, Taipei City, Taiwan 106 (CN); LEE, Shoeisheng, Taipei City, Taiwan 100 (CN)
(74) Representative: Casalonga, Axel
(86) International application number: PCT/CN2003/000477
(87) International publication number: WO 2004/110449

(57) **Abstract**

The invention provides certain aporphine and oxoaporphine for preparing medicine which can be used for ischemic disease prevention and treatment. The pharmacological mechanism of said compounds is to keep or increase endothelial nitric oxide synthase (eNOS), thus, the compounds can be used to produce medicines to prevent or treat mammal or human's ischemic disease, including ischemic apoplcxia, ischemic cerebral thrombosis, ischemic cerebral embolism, hypoxic-ischemic encephalopathy, ischemic heart disease or ischemic intestinal lesion.

## Description

### Field of the Invention

The present invention relates to a compound for treating ischemic diseases, more particularly to aporphine and oxoaporphine compound which has an endothelial nitric oxide synthase (eNOS) maintaining or enhancing action and thereby used to manufacture of a medicament for preventing or treating ischemic disease.

### Background of the Invention

With the progress of society and the advance in sciences and technology, the people has a longer life now than in the past. Many peoples suffer from disease because of the age, diet, obesity, deficiency of exercise or under a great living-stress, especially ischemic disease which is one of the main dead disease that may cause handicap. These will be a major impact or big loss to the people, family, society and the state, so it is very important to do some things to prevent the ischemic disease.

Among the ischemic disease, ischemic stroke has a higher morbidity, mortality, handicapping rate and recurrence rate, and it is a frequently encountered and common disease in the middle or old people. The occlusive lesion of the extracranial or intracranial arteries which provid blood for the brain may cause brain ischemia and hypoxemia, and a series of acute clinical symptoms (i.e. Ischemic stroke) will occur consequently. If blood supply can not be recovered in time, the nerve cells, glial cells and blood vessel will be necrotizing, and the cerebral infarction such as the cerebral thrombosis and cerebral embolism will emerge.

For the ischemic stroke, the thrombolytic agent(Tissue Plasminogen Activator) is the sole medicament which can "open up the blood vessel", which is also the sole medicament that has been approved to used for thromboembolic sroke by the Food and Drug Administration of USA, and it had also been approved by the Health Administration of Taiwan in 2001. However, patients may be subject to cerebral hemorrhagic complication after administrated the thrombolytic agent, so that the medicament is restricted strictly in its therapeutical time, i.e. "critical period", which means that the drug should be injected intravenously within 3 hours after stroke, or injected intra-arterially in combination with cerebral angiography to lysis the thrombus within 6 hours stroke. Other conventional "opening blood vessel" medicaments, such as anticoagulant and platelet inhibitor, can only used to prevent the thrombus from forming and increasing further, but can not lysis the infarcted thrombus to open up the blood vessel. In addition, brain tissue protectant(such as Piracetam) is a promising therapy for those diseases too, but it is preferably administrated within 6 hours after stroke so as to achieve a significant effect. The recent research shows that the thrombolytic agent can potentialize the information transmission of N-methyl-D-asparagic acid (NMDA) receptors so as to cause nerve cell died, and there are certain side effects to the patients, such as memory loss and body temperature descending etc., and the effect of thrombolytic-agent-therapy for the ischemic stroke is greatly decreased.

### Summary of the Invention

To efficiently overcome the above-described drawbacks of the art, the present inventor has developd a compound which has an endothelial nitric oxide synthase (eNOS) maintaining or enhancing action and thereby used to manufacture of a medicament for preventing or treating ischemic diseases.

The object of the invention is to provide an aporphine and oxoaporphine compound, the present compound has an endothelial nitric oxide synthase (eNOS) maintaining or enhancing action and thereby used to manufacture of a medicament for preventing or treating ischemic diseases.

To achieve the above-described purpose, the present invention provides an aporphine and oxoaporphine compound having the following structure I :

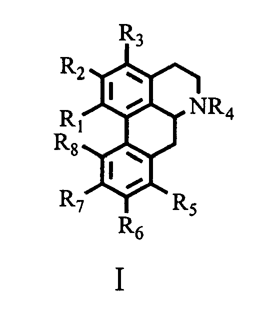

R1, R2, R6 and R7 are selected from H, OH, O-acyl, OMe, OEt, OⁿPr and OⁱPr, R3 and R5 are selected from H, OH, O-acyl, OMe, F, Cl, Br, NH₂, NO₂ or CN, R4 is selected from Allyl or CₙH2ₙ₊₁, n≥0, R8 is selected from H, OH, OMe.

The present invention provides an aporphine and oxoaporphine compound having the following structure II:

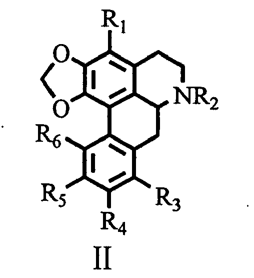

R1 and R3 are selected from H, OH, O-acyl, OMe, F, Cl, Br, NH₂, NO₂ or CN, R2 is selected from allyl or CₙH2ₙ₊₁, n≥0, R7 is selected from H, OH, OMe, R4 and R5 are selected from H, OH, O-acyl, OMe, OEt, OⁿPr, OⁱPr, R6 is selected from H, OH, O-acyl, OMe..

The present invention provides an aporphine and oxoaporphine compound having the following structure III : 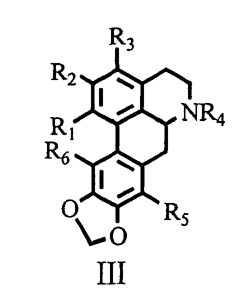 R1 and R2 are selected from H, OH, O-acyl, OMe, OEt, OⁿPr, OⁱPr, R3 and R5 are selected from H, OH, O-acyl, OMe, F, Cl, Br, NH₂, NO₂ or CN, R4 is selected from Allyl or CₙH2ₙ₊₁, n≥0, R6 is selected from H, OH, O-acyl, OMe.

The present invention provides an aporphine and oxoaporphine compound having the following structure IV :

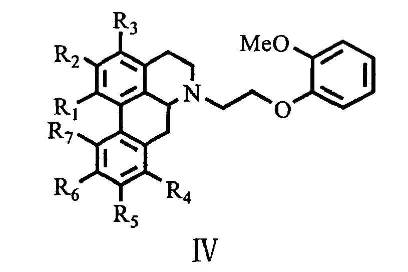

R1, R2, R5 and R6 are selected from H, OH, O-acyl, OMe, OEt, OⁿPr, OⁱPr, R3 and R4 are selected from H, OH, O-acyl, OMe, F, Cl, Br, NH₂, NO₂ or CN, R7 is selected from H, OH, O-acyl, OMe.

The present invention provides an aporphine and oxoaporphine compound having the following structure V :

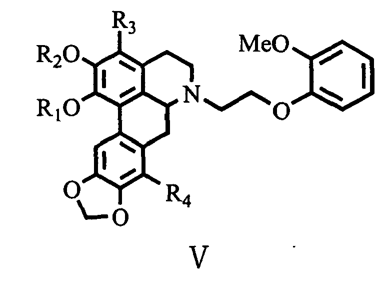

R1 and R2 are selected from H, acyl, Me, Et, OⁿPr, OⁱPr, R3 and R4 are selected from H, OH, O-acyl, OMe, F, Cl, Br, NH₂, NO₂ or CN.

The present invention provides an aporphine and oxoaporphine compound having the following structure VI :

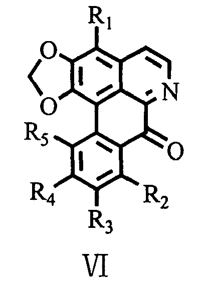

R1 and R2 are selected from H, OH, O-acyl, OMe, F, Cl, Br, NO₂ or CN, R3 and R4 are selected from H, OH, O-acyl, OMe, OEt, OⁿPr, OⁱPr, R5 is selected from H, OH, O-acyl, OMe.

The present invention provides an aporphine and oxoaporphine compound having the following structure VII :

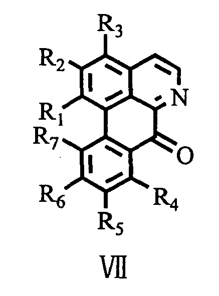

R1, R2, R5 and R6 are selected from H, OH, O-acyl, OMe, OEt, OⁿPr or OⁱPr, R3 and R4 are selected from H, OH, O-acyl, OMe, F, Cl, Br, NO₂ or CN, R7 is selected from H, OH, O-acyl, OMe.

The present invention provides an aporphine and oxoaporphine compound having the following structure VIII :

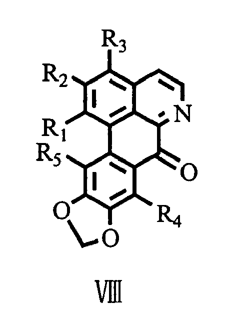

R1 and R2 are selected from H, OH, O-acyl, OMe, OEt, OⁿPr or OⁱPr, R3, and R4 are selected from H, OH, O-acyl, OMe, F, Cl, Br, NO₂ or CN, R5 is selected from H, OH, O-acyl, OMe.

The present compound can be used in manufacture of a medicament for treating ischemic disease in mammal or human being. More particularly, the ischemic disease includes ischemic cerebral apoplexy, ischemic cerebral thrombosis, ischemic cerebral embolism, hypoxia and ischemic encephlopathy, ischemic cardiac disease and ischemic enteropathy and the like.

The invention provides an aporphine and oxoaporphine compound used in manufacture of a medicament for preventing or treating ischemic disease. Compared with the current method in the art of lysising the infarcted thrombus to open blood vessel, the compound have a better therapeutic efficacy via loosening and dilatating the vessel. There are no side effects to the patients during treatment of the ischemic disease with the aporphine and oxoaporphine compound, said side effects such as memory loss, body temperature decreasing, and thus the effects will be excellent.

The present invention also disclosed the use of aporphine and oxoaporphine compound in the prophylaxis or treatment of ischemic disease, and further disclosed the use of aporphine and oxoaporphine compound in the prophylaxis or treatment of ischemic disease in mammal and human being.

The invention also provides a pharmaceutical composition for the prophylaxis or treatment of ischemic disease, comprising a therapeutically effective amount of aporphine and oxoaporphine compound and a pharmaceutically acceptable carrier or excipient.

The following examples further describe and demonstrate particular embodiments within the scope of the present invent. The examples are given solely for illustration and are not be construed as limitations.

### Brief Description of the Drawings

Fig.1 shows the comparative curve of the results between group I and II in the pharmacodynamic study.
Fig.2 shows the comparative]curve of the results between group I and III in the pharmacodynamic study.
Fig.3 shows effects of Liriodenine on eNOS protein and α-tubulin expression which have been ischemic for 30 min and followed by 2-hr reperfusion.
Fig.4 shows effects of Liriodenine on eNOS protein expression in human being umbilical cord vessel endothelium cells (HUVEC).

### Best Mode for Carrying out the Invention

Human being's blood vessel tissue can synthesize nitric oxide (NO) by itself, while NO will dilate blood vessel and thereby is in a close relation with blood pressure. The endogenous NO paly an important role in the vascular smooth muscle relaxation. In the *ex vivo* aortic ring and the topical vascular bed and general experiments, the vessel will constrict and the blood pressure will elevate if the formation of NO is been interrupted acutely. While in the mammal body, L-arginine is converted to L-citrulline and NO via an intermediate product in the presence of nitric oxide synthase (NOS) as catalyst, the NO formation process is as follow:

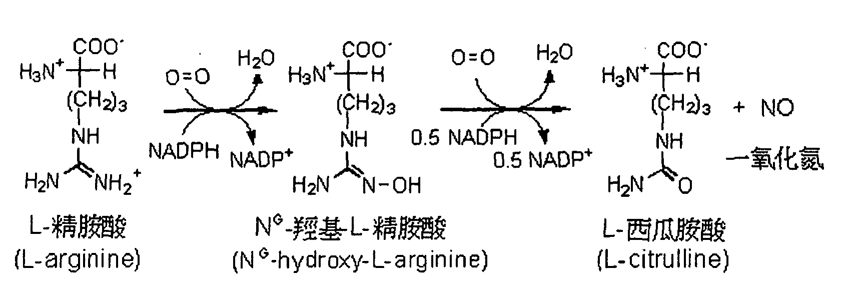

NOS exists in at least three isoforms, including neuronal NOS (nNOS, type I NOS), inducible NOS (iNOS or type II NOS) and endothelial NOS (eNOS or type III NOS). The function of eNOS is to adjust the vessel tension, while the function and mechanism of NO transferring information are different because of its origins. eNOS has three major functions: (1) used as nerve conduction factor in nerve synapse, and may contribute to brain learning and memory; (2) can relax the vascular smooth muscle in the vascular endothelium so as to dilate the vessel, and can descend blood pressure; and (3) can damage tumor cells to kill or cease their growth in the macrophage. Moreover, nNOS and eNOS are constitutive, i.e. calcium combine with calmodulin at first, then they combine with nNOS or eNOS to gain a atalytic activity; iNOS is inducible and is calcium-indepenent and calmodulin-indepenent, so cytokines can induce directly the catalysis of iNOS. Because iNOS is calcium-indepenent and calmodulin-indepenent, the reaction induced by iNOS can not be ceased and usually carry out for several hours and produce excessive NO, and then make the NO a harmful compound. Prior to this invention, cetain specific compounds which have a eNOS maintaining or enhancing action had been used to treat cardiovascular diseases (eg. arrhythmia, Su MJ, et al., Drug Development Research, 2001, 52:446-453), while the present inventers provide some new compounds used for preventing or treating ischemic disease such as stroke via the above-described mechanism.

The invention provides an aporphine and oxoaporphine compound used for preventing or treating ischemic diseases, and said compound has an endothelial nitric oxide synthase (eNOS) maintaining or enhancing action and thereby used to prevent or treat ischemic diseases. This compound may be Lirodenine, one of the formula VI compound that is used as a preferred example for demonstrating the present invention's effects. The structure VI is as follow:

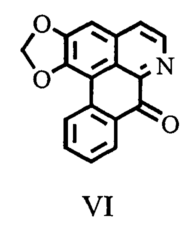

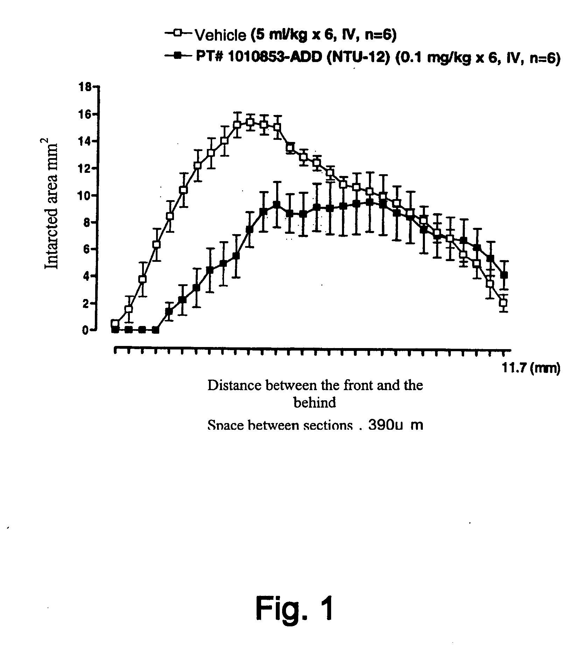

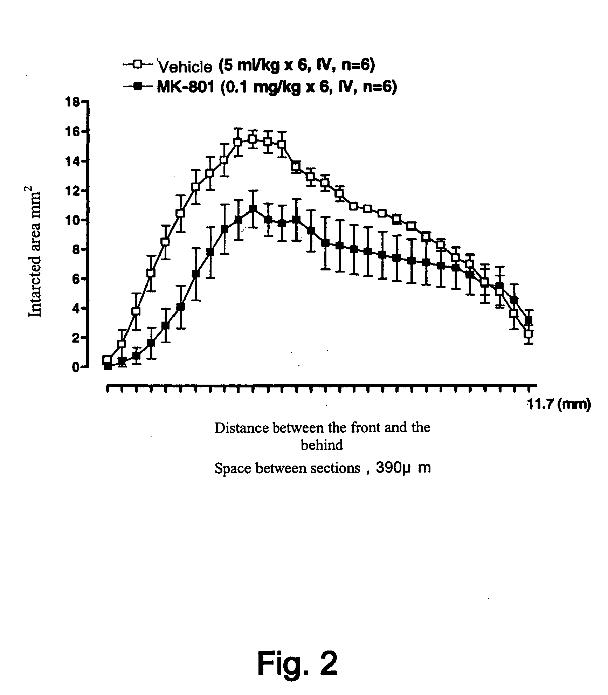

After Liriodenine is administrated to male Sprague Dawley rats, the effect of liriodenine on the artery occlusive cerebral ischemia in the rats is observed. First, the permanent brain ischemia is made via a middle cerebral artery occlusion (MCAO) in the rats; thereafter, at 0, 6, 24, 48, 48 and 54 hours later after the formation of middle cerebral artery occlusion (MCAO), Group I (i.e.vehicle control group) animals are given the solvent(0.9% NaCl)(5ml/kg, i.v.); Group II (PT#1010853-ADD(NTU-12)(NTU-106)) animals are given liriodenine(0.1mg/kg, i.v.); Group III animals are given MK-801(0.1mg/kg, i.v.), a blocking agent to the N-methyl-D-asparagic acid (NMDA) receptors. There are six rats in each group, the body temperature is measured via anal before and 15min later after administration. Four days later after the formation of MCAO, all the rats are killed, then brain section is made and stained with 2% cresol purple, the area and volume of cerebral ischemia lesions in each section is recorded, the results is showed in table 1, 2 and 3 respectively, the comparative results among these three groups are showed in table 4. The results can also be showed as graph, for example, Fig.1 shows the comparative curve of the results between group I and group II, and Fig.2 shows the comparative curve of the results between group I and group III. From these tables and figures, compared with MK-801, we could find the present compound exerted a more significant effect on reducing the area and volume of cerebral ischemia lesion during the treatment of ischemic apoplexy. The results of the body temperature is showed in table 5, 6 and 7. Compared with the results of group I, the present compound can not cause a greater changes in body temperature, while MK-801 cause side effect, i.e. body temperature descending.

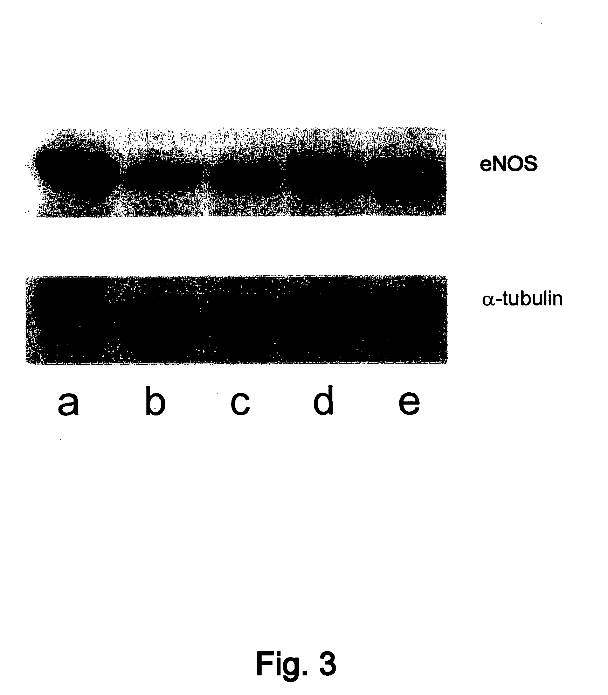

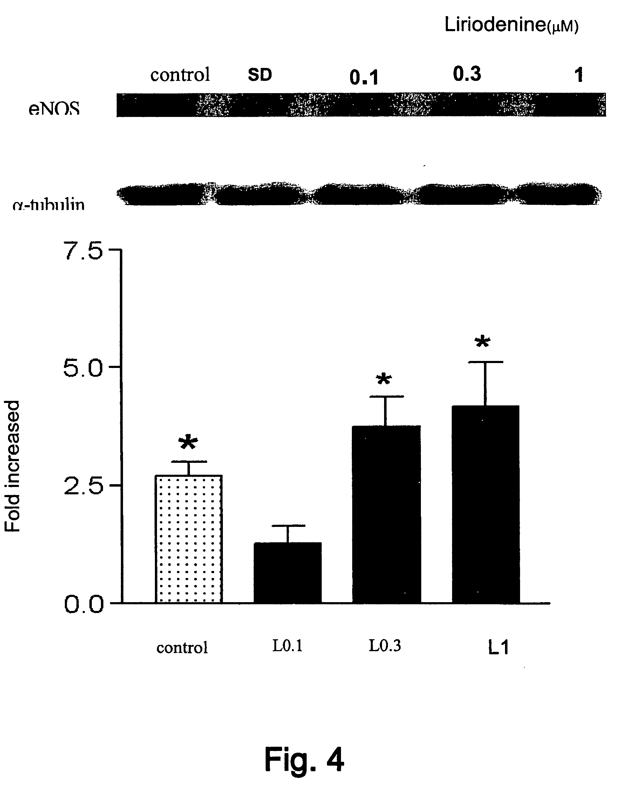

Fig.3 and Fig.4 demonstrated that liriodenine can protect eNOS or facilitate its formulation generation, and achieve the aim of preventing or treating ischemic disease thereby.

Fig.3 shows the effects of Liriodenine on eNOS protein expression which have been ischemic for 30 min and followed by 2-hr reperfusion, and the expression of α-tubulin indicates standard quantitative index. In the figure, Fig.a shows the effect on eNOS and α-tubulin expression in the heart of male rats under normal condition; Fig.b shows the effect on eNOS and α-tubulin expression in non-infarcted area, wherein the left anterior descending branch coronary artery(LAD) of the heart has been ligated and followed by perfusion with vehicle-containing solution; Fig.c shows the effect on eNOS and α-tubulin expression in infarcted area, wherein the left anterior descending branch coronary artery of the heart has been ligated and followed by perfusion with vehicle effect on eNOS and α -tubulin expression in non-infarcted area, wherein the left anterior descending branch coronary artery of the heart has been ligated and followed by perfusion with 1µM liriodenine; and Fig.e shows the effect on eNOS and α-tubulin expression in infarcted area, wherein the left anterior descending branch coronary artery of the heart has been ligated and followed by perfusion with 1µM liriodenine difference in expression between eNOS and α-tubulin, we conclude that the expression of eNOS in the heart can approach the normal value after perfused with liriodenine solution, these results demonstrated sufficiently that the present compound (such as liriodenine)can improve the expression of eNOS or maintain its expression quantity at a constant level.

Fig.4 shows the effects of Liriodenine on eNOS protein expression in human being umbilical cord vessel endothelium cells (HUVEC) wherein blood serum have been removed. There is a segregation distorter (SD) phenomenon in this figure, said SD refers to a chromatosome appears in the natural family or cluster. The increasing progression of related protein is quantified using density -image software, the results showed in the figure are representative curves of three separate tests with the same results, the quantity of HUVEC eNOS without blood serum is defined as 1(wherein the expression quantity of α-tubulin indicates standard quantitative index), and "P<0.05" means that the difference is significant from the quantity of HUVEC eNOS without blood serum. From the results in Fig.4, we can find that quantity of eNOS increase significantly after the HUEVC is treated with 0.3 and 1µM of lirodenine, this result demonstrates that lirodenine exertes an effect on maintaining eNOS or facilitating the formation of eNOS.

Therefore, the invention can maintain the function of eNOS via a mechanism of increasing eNOS, said functions include inhibit leucocyte and platelet adhesion, as well as adjust the blood tension etc. The individual effect on the cerebral ischemia is confirmed in the transgenic animal model. If there is topical cerebral ischemia, the infarction will be larger in eNOS gene-knockout rats, while the infarction in rats without eNOS gene decrease significantly compared with that in the wide type rats, and the infarction of eNOS gene-knockout 2 rats are small too. These results demonstrated that the invention can decrease the volume and area of cerebral ischemia in male rats without side effects, such as those side effects caused by MK801 (a blocking agent to the NMDA receptor).

In addition, the compound described above not only can treat ischemic disease in male rat, but also treat the same disease in the mammal and human being. The said ischemic diseases include such as ischemic cerebral thrombosis, ischemic cerebral embolism, hypoxic ischemic encephlopathy disease, ischemic cardiac disease or ischemic enteropathy, as well as ischemic cerebral apoplexy.

| **Table 1 (Summary)** | | | | | |
|---|---|---|---|---|---|
| Calculated Decrease of Test Compound in the Total Volume of Ischemic Lesions in the Brains of MCAO Rats | | | | | |
| Treatment | Route | Dose | N | Infarcted Volume (X±SEM mm³) | % Inh. (X±SEM) |
| Vehicle Control | IV | 5ml/kg×6 | 6 | 109.92±2.96 | - |
| PT#1010853-ADD | IV | 0.1mg/kg×6 | 6 | 71.37±11.87* | 35.07±10.80 |
| (NTU-12) | | | | | |
| (NTU-106) | | | | | |
| MK-801 | IV | 0.1mg/kg×6 | 6 | 75.17±13.74* | 31.61±12.50 |

| | | | | | |
|---|---|---|---|---|---|
| *P<0.05 | | | | | |

| Table 2 | | | | | | | |
|---|---|---|---|---|---|---|---|
| Area of Ischemic Lesions in Vehicle Control (0.9% NaCl, 5 ml/kg × 6, IV) MCAO Rats | | | | | | | |
| Slice# | Infarcted Area (mm²) of Each Tested Animal | | | | | | X±SEM |
| | 1 | 2 | 3 | 4 | 5 | 6 | N=6 |
| BW(g) | 250 | 240 | 240 | 250 | 260 | 270 | |
| | | | | | | | |
| 1 | 1.52 | 0 | 0 | 0 | 1.20 | 0 | 0.45±0.29 |
| 2 | 5.05 | 0 | 0 | 0 | 4.20 | 0 | 1.54±0.98 |
| 3 | 8.50 | 1.18 | 3.16 | 0 | 5.11 | 4.57 | 3.75±1.24 |
| 4 | 10.80 | 4.50 | 4.62 | 2.68 | 8.25 | 7.17 | 6.34±1.21 |
| 5 | 12.60 | 8.71 | 6.25 | 4.44 | 9.45 | 9.28 | 8.45±1.15 |
| 6 | 15.00 | 9.38 | 8.68 | 6.49 | 13.00 | 9.97 | 10.42±1.26 |
| 7 | 15.90 | 11.00 | 9.67 | 9.23 | 15.30 | 12.30 | 12.23±1.15 |
| 8 | 16.00 | 12.00 | 10.80 | 10.30 | 16.90 | 13.00 | 13.17±1.11 |
| 9 | 17.00 | 12.70 | 11.50 | 12.20 | 17.90 | 13.10 | 14.07±1.10 |
| 10 | 17.60 | 14.20 | 11.60 | 15.30 | 17.60 | 15.30 | 15.27±0.92 |
| 11 | 16.60 | 15.60 | 12.70 | 15.70 | 16.60 | 15.60 | 15.47±0.59 |
| 12 | 15.80 | 13.70 | 12.80 | 15.80 | 15.90 | 17.70 | 15.28±0.72 |
| 13 | 13.50 | 13.60 | 13.00 | 16.80 | 15.70 | 18.10 | 15.12±0.85 |
| 14 | 13.30 | 12.60 | 12.70 | 14.80 | 13.40 | 14.70 | 13.58±0.39 |
| 15 | 11.60 | 11.70 | 11.70 | 14.40 | 13.30 | 14.70 | 12.90±0.58 |
| 16 | 11.50 | 11.10 | 11.50 | 14.20 | 12.70 | 13.90 | 12.48±0.54 |
| 17 | 11.20 | 11.00 | 11.30 | 14.10 | 12.20 | 10.80 | 11.77±0.51 |
| 18 | 10.00 | 10.90 | 11.20 | 11.80 | 10.70 | 10.80 | 10.90±0.24 |
| 19 | 9.97 | 10.80 | 11.10 | 11.50 | 10.50 | 10.40 | 10.71±0.22 |
| 20 | 9.96 | 10.70 | 11.00 | 11.00 | 9.82 | 9.90 | 10.40±0.23 |
| 21 | 9.70 | 10.60 | 10.30 | 11.00 | 8.73 | 9.80 | 10.02±0.33 |
| 22 | 9.60 | 9.72 | 9.76 | 10.50 | 8.68 | 8.97 | 9.54±0.26 |
| 23 | 8.77 | 8.80 | 9.50 | 9.60 | 7.47 | 8.75 | 8.81±0.31 |
| 24 | 8.74 | 8.44 | 8.74 | 8.86 | 6.30 | 8.35 | 8.24±0.40 |
| 25 | 8.38 | 8.31 | 7.83 | 8.60 | 3.95 | 7.10 | 7.36±0.72 |
| 26 | 7.96 | 7.73 | 7.12 | 8.38 | 3.82 | 6.55 | 6.93±0.67 |
| 27 | 5.81 | 7.70 | 6.82 | 6.45 | 1.59 | 6.06 | 5.74±0.87 |
| 28 | 5.73 | 7.41 | 6.62 | 5.87 | 0 | 4.96 | 5.10±1.07 |
| 29 | 5.00 | 6.71 | 4.95 | 4.00 | 0 | 0.94 | 3.60±1.06 |
| 30 | 3.09 | 2.61 | 3.83 | 3.11 | 0 | 0.58 | 2.20±0.63 |
| Totalmm² | 316.18 | 273.40 | 260.75 | 277.11 | 280.27 | 283.35 | 281.84±7.58 |
| **Total**mm³ | 123.31 | 106.63 | 101.69 | 108.07 | 109.31 | 110.51 | 109.92±2.96 |
| %Inh. | | | | | | | |

| **Table 3** | | | | | | | |
|---|---|---|---|---|---|---|---|
| Area of Ischemic Lesions in PT#1010853-ADD (NTU-12)(NTU-106)(0.1mg/kg×6,IV) MCAO Rats | | | | | | | |
| Slice# | Infarcted Area (mm²) of Each Tested Animal | | | | | | X±SEM |
| | 1 | 2 | 3 | 4 | 5 | 6 | N=6 |
| BW(g) | 230 | 240 | 240 | 250 | 240 | 230 | |
| | | | | | | | |
| 1 | 0 | 0 | 0 | 0 | 0 | 0 | 0 |
| 2 | 0 | 0 | 0 | 0 | 0 | 0 | 0 |
| 3 | 0 | 0 | 0 | 0 | 0 | 0 | 0 |
| 4 | 0 | 0 | 0 | 0 | 0 | 0 | 0 |
| 5 | 0 | 3.86 | 2.61 | 0 | 0 | 1.97 | 1.41±0.68 |
| 6 | 0 | 6.38 | 4.31 | 0 | 0 | 2.95 | 2.27±1.11 |
| 7 | 0.93 | 8.47 | 5.33 | 0 | 0 | 4.30 | 3.17±1.40 |
| 8 | 3.20 | 8.55 | 6.94 | 0 | 0 | 8.17 | 4.48±1.61 |
| 9 | 4.52 | 9.10 | 7.91 | 0 | 0.61 | 7.68 | 4.97±1.60 |
| 10 | 4.73 | 9.11 | 9.92 | 0 | 2.44 | 7.10 | 5.55±1.58 |
| 11 | 8.54 | 10.30 | 10.50 | 1.75 | 6.97 | 7.09 | 7.53±1.31 |
| 12 | 9.95 | 12.60 | 10.90 | 2.80 | 10.70 | 6.22 | 8.86±1.49 |
| 13 | 10.60 | 12.00 | 13.80 | 3.44 | 11.30 | 5.23 | 9.39±1.67 |
| 14 | 10.70 | 11.20 | 10.71 | 3.56 | 11.40 | 4.93 | 8.75±1.44 |
| 15 | 11.50 | 10.70 | 9.99 | 3.84 | 12.54 | 3.67 | 8.71±1.60 |
| 16 | 12.10 | 10.60 | 9.83 | 5.92 | 14.30 | 2.48 | 9.20±1.76 |
| 17 | 12.40 | 10.50 | 9.70 | 6.59 | 14.50 | 1.17 | 9.14±1.93 |
| 18 | 13.30 | 10.40 | 9.68 | 7.33 | 15.10 | 0 | 9.30±2.17 |
| 19 | 13.90 | 10.40 | 9.55 | 7.37 | 15.70 | 0 | 9.49±2.26 |
| 20 | 14.80 | 9.91 | 9.10 | 9.30 | 14.60 | 0 | 9.62±2.20 |
| 21 | 15.20 | 9.31 | 9.08 | 8.56 | 14.29 | 0 | 9.41±2.21 |
| 22 | 14.20 | 9.14 | 8.92 | 8.13 | 12.50 | 0 | 8.82±2.01 |
| 23 | 13.90 | 9.01 | 8.85 | 7.79 | 11.44 | 0 | 8.50±1.92 |
| 24 | 12.10 | 7.90 | 6.89 | 7.45 | 11.10 | 0 | 7.57±1.74 |
| 25 | 10.50 | 7.86 | 6.71 | 6.76 | 11.00 | 0 | 7.14±1.61 |
| 26 | 10.40 | 7.80 | 6.50 | 6.23 | 10.70 | 0 | 6.94±1.59 |
| 27 | 10.30 | 7.68 | 6.05 | 6.16 | 10.60 | 0 | 6.80±1.58 |
| 28 | 9.74 | 6.98 | 5.48 | 6.08 | 9.27 | 0 | 6.26±1.43 |
| 29 | 8.03 | 5.88 | 4.80 | 5.35 | 8.79 | 0 | 5.47±1.27 |
| 30 | 7.94 | 4.30 | 4.40 | 3.95 | 4.98 | 0 | 4.26±1.04 |
| **Total**mm² | 243.48 | 229.94 | 208.46 | 118.36 | 234.83 | 62.96 | 183.00±30.43 |
| **Total**mm³. | 94.96 | 89.68 | 81.30 | 46.16 | 91.58 | 24.55 | 71.37±11.87* |
| %Inh | 13.61 | 18.41 | 26.04 | 58.01 | 16.68 | 77.67 | 35.07±10.80 |

| Table 4 | | | | | | | |
|---|---|---|---|---|---|---|---|
| Area of Ischemic Lesions in MK-801(0.1mg/kg×6,IV) MCAO Rats | | | | | | | |
| Slice# | Infarcted Area (mm²) of Each Tested Animal | | | | | | X±SEM |
| | 1 | 2 | 3 | 4 | 5 | 6 | N=6 |
| BW(g) | 220 | 250 | 250 | 260 | 240 | 270 | |
| | | | | | | | |
| 1 | 0 | 0 | 0 | 0 | 0 | 0 | 0 |
| 2 | 0 | 1.91 | 0 | 0 | 0 | 0 | 0.32±0.32 |
| 3 | 0 | 3.53 | 0 | 0 | 0 | 0.97 | 0.75±0.58 |
| 4 | 0 | 5.86 | 0 | 0 | 0 | 3.76 | 1.60±1.05 |
| 5 | 1.99 | 6.74 | 0 | 0 | 2.52 | 5.58 | 2.81±1.15 |
| 6 | 4.33 | 8.02 | 0 | 0 | 4.32 | 7.78 | 4.08±1.44 |
| 7 | 5.86 | 10.50 | 2.74 | 0 | 7.54 | 11.10 | 6.29±1.78 |
| 8 | 6.97 | 11.90 | 5.11 | 1.18 | 9.83 | 11.70 | 7.78±1.71 |
| 9 | 8.63 | 12.30 | 6.39 | 2.98 | 13.90 | 12.00 | 9.37±1.70 |
| 10 | 10.60 | 12.80 | 7.72 | 4.32 | 12.20 | 12.40 | 10.01±1.37 |
| 11 | 12.80 | 14.00 | 8.21 | 5.78 | 12.00 | 11.70 | 10.75±1.27 |
| 12 | 12.90 | 12.20 | 8.81 | 5.24 | 10.70 | 10.20 | 10.01±1.12 |
| 13 | 13.90 | 10.80 | 8.93 | 4.73 | 10.10 | 10.10 | 9.76±1.22 |
| 14 | 15.00 | 10.80 | 10.10 | 4.04 | 10.10 | 9.94 | 10.00±1.43 |
| 15 | 12.80 | 10.70 | 9.59 | 2.64 | 10.00 | 9.74 | 9.24±1.41 |
| 16 | 12.80 | 10.20 | 8.70 | 0 | 9.84 | 8.83 | 8.40±1.78 |
| 17 | 12.70 | 9.82 | 8.20 | 0 | 9.66 | 8.82 | 8.20±1.76 |
| 18 | 12.00 | 9.65 | 8.11 | 0 | 9.10 | 8.81 | 7.95±1.68 |
| 19 | 11.90 | 9.42 | 7.78 | 0 | 9.05 | 8.68 | 7.81±1.66 |
| 20 | 11.30 | 9.35 | 7.74 | 0 | 9.01 | 8.03 | 7.57±1.60 |
| 21 | 10.70 | 9.08 | 7.66 | 0 | 8.67 | 8.00 | 7.35±1.53 |
| 22 | 10.10 | 9.04 | 7.31 | 0 | 8.60 | 7.92 | 7.16±1.48 |
| 23 | 10.10 | 8.81 | 7.22 | 0 | 8.45 | 7.76 | 7.06±1.47 |
| 24 | 9.65 | 8.68 | 6.80 | 0 | 8.16 | 7.72 | 6.84±1.42 |
| 25 | 9.64 | 8.44 | 6.65 | 0 | 8.14 | 7.30 | 6.69±1.40 |
| 26 | 8.88 | 8.01 | 5.95 | 0 | 7.66 | 6.87 | 6.23±1.31 |
| 27 | 8.45 | 7.77 | 3.80 | 0 | 7.33 | 6.40 | 5.63±1.30 |
| 28 | 8.44 | 7.68 | 3.47 | 0 | 7.17 | 6.00 | 5.46±1.30 |
| 29 | 7.98 | 5.38 | 3.09 | 0 | 5.31 | 5.31 | 4.51±1.10 |
| 30 | 3.89 | 5.02 | 3.03 | 0 | 4.08 | 2.94 | 3.16±0.70 |
| **Total**mm² | 254.31 | 258.41 | 163.11 | 30.91 | 223.44 | 226.36 | 192.76±35.23 |
| **Total**Mm³ | 99.18 | 100.78 | 63.61 | 12.05 | 87.14 | 88.28 | 75.17±13.74* |
| %Inh. | 9.77 | 8.32 | 42.13 | 89.04 | 20.72 | 19.69 | 31.61±12.50 |

| Table 5 | | | | | | | | |
|---|---|---|---|---|---|---|---|---|
| Body Temperature (°C) At 0 Minute Before (Pre) And 15 Min After(Post) Compound reatment | | | | | | | | |
| Treatment | Route | N | 0 Hour Dosage | | | 6 Hours Dosage | | |
| | | | Pre | Post | Δ | Pre | Post | Δ |
| Vehicle (5ml/kg) | IV | 1 | 37.9 | 36.0 | -1.9 | 37.4 | 37.9 | 0.5 |
| | IV | 2 | 38.1 | 36.8 | -1.3 | 37.6 | 38.4 | 0.8 |
| | IV | 3 | 37.9 | 36.5 | -1.4 | 37.3 | 38.0 | 0.7 |
| | IV | 4 | 38.4 | 37.8 | -0.6 | 38.1 | 36.4 | -1.7 |
| | IV | 5 | 38.1 | 38.7 | 0.6 | 37.3 | 38.0 | 0.7 |
| | IV | 6 | 37.5 | 36.1 | -1.4 | 37.9 | 37.8 | -0.1 |
| | X | | 38.0 | 37.0 | -1.0 | 37.6 | 37.8 | 0.1 |
| | SEM | | 0.1 | 0.4 | 0.4 | 0.1 | 0.3 | 0.4 |

| Treatment | Route | N | 24 Hours Dosage | | | 30 Hours Dosage | | |
|---|---|---|---|---|---|---|---|---|
| | | | Pre | Post | Δ | Pre | Post | Δ |
| Vehicle (5ml/kg) | IV | 1 | 38.3 | 38.0 | -0.3 | 37.7 | 37.2 | -0.5 |
| | IV | 2 | 38.1 | 37.9 | -0.2 | 37.7 | 38.2 | 0.5 |
| | IV | 3 | 37.6 | 36.2 | -1.4 | 37.9 | 37.5 | -0.4 |
| | IV | 4 | 38.2 | 37.5 | -0.7 | 37.3 | 37.9 | 0.6 |
| | IV | 5 | 37.9 | 37.3 | -0.6 | 38.1 | 37.8 | -0.3 |
| | IV | 6 | 38.0 | 37.9 | -0.1 | 37.6 | 38.0 | 0.4 |
| | X | | 38.0 | 37.5 | -0.5 | 37.7 | 37.8 | 0.1 |
| | SEM | | 0.1 | 0.3 | 0.2 | 0.1 | 0.1 | 0.2 |

| Treatment | Route | N | 48 Hours Dosage | | | 54 Hours Dosage | | |
|---|---|---|---|---|---|---|---|---|
| | | | Pre | Post | Δ | Pre | Post | Δ |
| Vehicle (5ml/kg) | IV | 1 | 37.9 | 37.0 | -0.9 | 37.9 | 38.0 | 0.1 |
| | IV | 2 | 38.0 | 37.5 | -0.5 | 37.6 | 37.9 | 0.3 |
| | IV | 3 | 37.4 | 38.0 | 0.6 | 38.2 | 38.1 | -0.1 |
| | IV | 4 | 37.3 | 38.5 | 1.2 | 38.7 | 39.0 | 0.3 |
| | IV | 5 | 37.1 | 37.7 | 0.6 | 38.5 | 38.3 | -0.2 |
| | IV | 6 | 37.6 | 37.5 | -0.1 | 37.7 | 38.0 | 0.3 |
| | X | | 37.6 | 37.7 | 0.2 | 38.1 | 38.2 | 0.1 |
| | SEM | | 0.1 | 0.2 | 0.3 | 0.2 | 0.2 | 0.1 |

| **Table 6** | | | | | | | | |
|---|---|---|---|---|---|---|---|---|
| Body Temperature (°C) At 0 Minute Before (Pre) And 15 Min After(Post) Compound Treatment | | | | | | | | |
| Treatment | Route | N | 0 Hour Dosage | | | 6 Hours Dosage | | |
| | | | Pre | Post | Δ | Pre | Post | Δ |
| PT#1010853-ADD (NTU-12) (NTU-106) (0.1mg/kg×6) | IV | 1 | 37.0 | 36.3 | -0.7 | 37.8 | 38.3 | 0.5 |
| | IV | 2 | 38.0 | 37.1 | -0.9 | 37.9 | 38.5 | 0.6 |
| | IV | 3 | 37.0 | 37.6 | 0.6 | 38.3 | 38.3 | 0 |
| | IV | 4 | 36.9 | 37.9 | 1.0 | 38.2 | 38.6 | 0.4 |
| | IV | 5 | 37.4 | 37.1 | -0.3 | 38.1 | 37.8 | -0.3 |
| | IV | 6 | 37.5 | 36.9 | -0.6 | 37.5 | 38.4 | 0.9 |
| | X | | 37.3 | 37.1 | -0.1 | 38.0 | 38.3 | 0.3 |
| | SEM | | 0.2 | 0.2 | 0.3 | 0.1 | 0.1 | 0.2 |

| Treatment | Route | N | 24 Hours Dosage | | | 30 Hours Dosage | | |
|---|---|---|---|---|---|---|---|---|
| | | | Pre | Post | Δ | Pre | Post | Δ |
| PT#1010853-ADD | IV | 1 | 37.6 | 36.8 | -0.8 | 38.5 | 37.9 | -0.6 |
| (NTU-12) | IV | 2 | 38.6 | 38.2 | -0.6 | 37.7 | 37.7 | 0 |
| (NTU-106) (0.1mg/kg×6) | IV | 3 | 38.1 | 37.9 | -0.2 | 37.9 | 38.3 | 0.4 |
| | IV | 4 | 38.0 | 37.8 | -0.2 | 38.9 | 38.4 | -0.5 |
| | IV | 5 | 38.0 | 37.7 | -0.3 | 38.3 | 38.0 | -0.3 |
| | IV | 6 | 37.8 | 38.0 | 0.2 | 38.0 | 37.9 | -0.1 |
| | X | | 38.0 | 37.7 | -0.3 | 38.2 | 38.1 | -0.2 |
| | SEM | | 0.1 | 0.2 | 0.1 | 0.2 | 0.1 | 0.1 |

| Treatment | Route | N | 48 Hours Dosage | | | 54 Hours Dosage | | |
|---|---|---|---|---|---|---|---|---|
| | | | Pre | Post | Δ | Pre | Post | Δ |
| PT#1010853-ADD (NTU-12) (NTU-106) (0.1mg/kg×6) | IV | 1 | 37.9 | 37.5 | -0.4 | 37.9 | 38.2 | 0.3 |
| | IV | 2 | 38.2 | 38.0 | -0.2 | 37.5 | 38.0 | 0.5 |
| | IV | 3 | 38.2 | 37.6 | -0.6 | 38.0 | 37.6 | -0.4 |
| | IV | 4 | 38.5 | 38.1 | -0.4 | 38.0 | 38.2 | 0.2 |
| | IV | 5 | 37.7 | 38.0 | 0.3 | 37.4 | 37.8 | 0.4 |
| | IV | 6 | 38.2 | 38.0 | -0.2 | 37.7 | 38.0 | 0.3 |
| | X | | 38.1 | 37.9 | -0.3 | 37.7 | 38.0 | 0.2 |
| | SEM | | 0.1 | 0.1 | 0.1 | 0.1 | 0.1 | 0.1 |

| Table 7 | | | | | | | | |
|---|---|---|---|---|---|---|---|---|
| Body Temperature (°C) At 0 Minute Before (Pre) And 15 Min After(Post) Compound Treatment | | | | | | | | |
| Treatment | Route | N | 0 Hour Dosage | | | 6 Hours Dosage | | |
| | | | Pre | Post | Δ | Pre | Post | Δ |
| MK-801 (0.1mg/kg×6) | IV | 1 | 39.0 | 36.5 | -2.5 | 38.1 | 38.6 | 0.5 |
| | IV | 2 | 38.3 | 35.9 | -2.4 | 37.8 | 37.5 | -0.3 |
| | IV | 3 | 37.9 | 35.4 | -2.5 | 37.7 | 37.7 | 0 |
| | IV | 4 | 38.7 | 36.1 | -2.6 | 37.8 | 37.7 | -0.1 |
| | IV | 5 | 36.8 | 36.0 | -0.8 | 37.7 | 37.3 | -0.4 |
| | IV | 6 | 37.1 | 35.6 | -1.5 | 38.0 | 38.7 | 0.7 |
| | X | | 38.0 | 35.9 | -2.0 | 37.8 | 37.9 | 0.1 |
| | SEM | | 0.4 | 0.2 | 0.3 | 0.1 | 0.2 | 0.2 |

| Treatment | Route | N | 24 Hours Dosage | | | 30 Hours Dosage | | |
|---|---|---|---|---|---|---|---|---|
| | | | Pre | Post | Δ | Pre | Post | Δ |
| MK-801 (0.1mg/kg×6) | IV | 1 | 37.7 | 38.2 | 0.5 | 37.0 | 37.2 | 0.2 |
| | IV | 2 | 37.5 | 37.2 | 0.3 | 37.7 | 38.0 | 0.3 |
| | IV | 3 | 37.6 | 37.5 | -0.1 | 37.3 | 38.2 | 0.9 |
| | IV | 4 | 37.8 | 38.0 | 0.2 | 37.9 | 37.9 | 0 |
| | IV | 5 | 37.9 | 37.3 | -0.6 | 38.1 | 37.5 | -0.6 |
| | IV | 6 | 37.3 | 36.9 | -0.4 | 37.4 | 38.2 | 0.8 |
| | X | | 37.8 | 37.5 | 0 | 37.6 | 37.8 | 0.3 |
| | SEM | | 0.1 | 0.2 | 0.2 | 0.2 | 0.2 | 0.2 |

| Treatment | Route | N | 48 Hours Dosage | | | 54 Hours Dosage | | |
|---|---|---|---|---|---|---|---|---|
| | | | Pre | Post | Δ | Pre | Post | Δ |
| MK-801 (0.1mg/kg×6) | IV | 1 | 38.0 | 37.3 | -0.7 | 37.8 | 38.5 | 0.7 |
| | IV | 2 | 38.0 | 37.3 | -0.7 | 37.5 | 38.1 | 0.6 |
| | IV | 3 | 37.5 | 37.2 | -0.3 | 37.4 | 38.0 | 0.6 |
| | IV | 4 | 37.7 | 38.4 | 0.7 | 37.9 | 39.0 | 1.1 |
| | IV | 5 | 37.7 | 38.0 | 0.3 | 37.7 | 37.6 | -0.1 |
| | IV | 6 | 37.3 | 36.9 | -0.4 | 37.7 | 36.8 | -0.9 |
| | X | | 37.7 | 37.5 | -0.2 | 37.7 | 38.0 | 0.3 |
| | SEM | | 0.1 | 0.2 | 0.2 | 0.1 | 0.3 | 0.3 |

In addition, the above-mentioned formula VI compound for preventing or treating ischemic disease via the action of maintaining or improving eNOS,

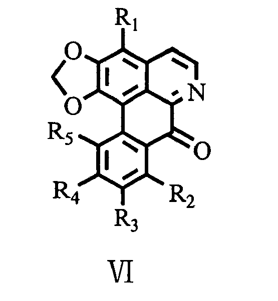

R1, R2, R3, R4 and R5 designate H, wherein R1 and R2 can be further selected from OH, OMe, F, C1, Br, NO₂ or CN; R3 and R4 can be OH, OEt, OⁿPr, OⁱPr or O-acyl; R5 can be OH or OMe, all the compound as described above can be used to prevent or treat the ischemic disease in the human being or other mammals.

Besides the above-mentioned formula VI compound, the compound for preventing or treating ischemic diseases may be the formula I compound:

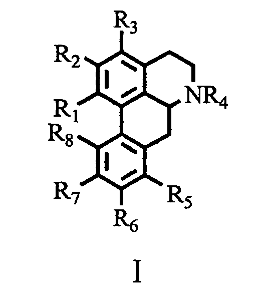

R1, R2, R6, and R7 are selected from H, OH, O-acyl, OMe, OEt, OⁿPr or OⁱPr; R3 and R5 are selected from H, OH, O-acyl, OMe, F, Cl, Br, NH₂, NO₂ or CN; R4 indicated as allyl or CₙH₂ₙ₊₁, n≥0; R8 designates H, OH, or OMe.

The compound for preventing or treating ischemic diseases may be the formula II compound:

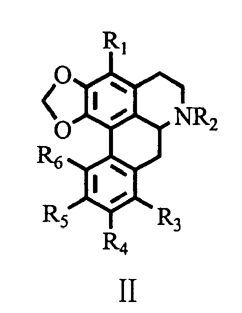

R1 and R3 are selected from H, OH, O-acyl, OMe, F, Cl, Br, NH₂, NO₂ or CN; R2 designates allyl or CₙH₂ₙ₊₁, n≥0; R4 and R5 are selected from H, OH, O-acyl, OMe, OEt, OⁿPr or OⁱPr; R6 designates H, OH, O-acyl, or OMe etc.

The compound for preventing or treating ischemic diseases may be the formula III compound:

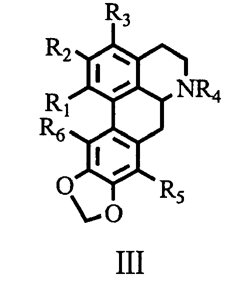

R1 and R2 are selected from H, OH, O-acyl, OMe, OEt, OⁿPr or OⁱPr; R3 and R5 are selected from H, OH, O-acyl, OMe, Cl, Br, NH₂, NO₂ or CN; R4 designates allyl or CₙH₂ₙ₊₁, n≥0; R6 designates H, OH, O-acyl, or OMe.

The compound for preventing or treating ischemic diseases may be the formula IV compound:

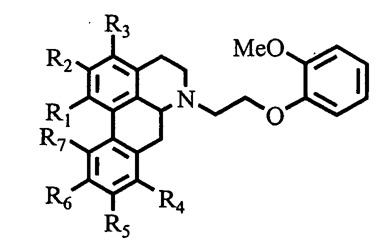

R1, R2, R5 and R6 are selected from H, OH, O-acyl, OMe, OEt, OⁿPr, OⁱPr; R4 designates H, OH, O-acyl, OMe, F, Cl, Br, NH₂, NO₂ or CN; R7 designates H, OH, O-acyl, or OMe.

The compound for preventing or treating ischemic diseases may be the formula V compound:

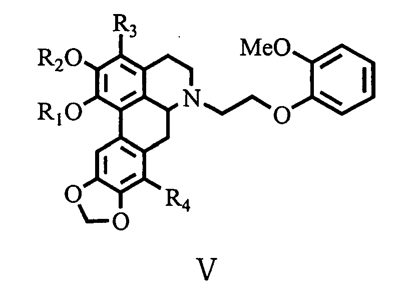

R1 and R2 are selected from H, acyl, Me, Et, ⁿPr or ⁱPr; R3 and R4 are selected from H, OH, O-acyl, OMe, F, Cl, Br, NH₂, NO₂ or CN.

The compound for preventing or treating ischemic diseases may be the formula VII compound:

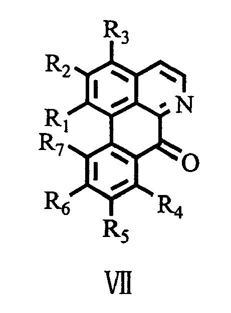

R1, R2, R5 and R6 are selected from H, OH, OAc, OMe, OEt, OⁿPr or OⁱPr; R3, R4 designate H, OH, O-acyl, OMe, F, Cl, Br, NO₂ or CN; R7 designates H, O-acyl, or OMe.

The compound for preventing or treating ischemic diseases may be the formula VIII compound:

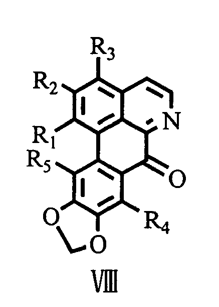

R1 and R2 designate H, OH, O-acyl, OMe, OEt, OⁿPr or OⁱPr; R3 and R4 are selected from H, OH, O-acyl, OMe, F, Cl, Br, NO₂ or CN; R5 designates H, OH, O-acyl, or OMe.

Any of the above-mentioned formula I to VIII compound can present in combination with a pharmaceutical acceptable carrier or excipient, and generally the said carrier or excipient is lactose.

All the aporphines and oxoaporphines compound with different structures as described above can be used to treat ischemic diseases in mammal or human being, and the ischemic disease include ischemic cerebra apoplexy, ischemic cerebral thrombosis, ischemic cerebral embolism, hypoxic ischemic encephlopathy, ischemic cardiac disease or ischemic enteropathy etc.

The following examples are provided for the purpose of further describing the preparation of the present aporphine and oxoaporphine compound.

### Example 1

### Preparation of 2,9-Diisopropyloxy-1,10-dimethoxy-7-oxoaporphine(3), the formula VII compound, wherein R1=R6=OMe , R2=R5=OⁱPr , R3=R4=R7=H; and 9-Hydroxy-2-isopropyloxy-1,10-dimethoxy-7-oxoaporphine(4), the formula VII compound, wherein R1=R6=OMe, R2=OⁱPr, R3=R4=R7=H, R5=OH.

1. **Preparation of 2,9-Diisopropyloxy-1,10-dimethoxy-N-methylaporphine(2),** the formula VI compound, wherein R1=R7=OMe, R2=R6=OiPr, R3=R5=R8=H, R4=Me.
   Boldine(1,1.63g, 5mmol), anhydrous alcohol (50ml), and anhydrous potassium carbonate(3.0g) are added into a round bottom flask in turn while stiring in the oil bath(70°C), then adding dropwise iodide propane (3.4g, 20 ml) in anhydrous alcohol solution(10ml) over 1 hour.The reaction is reacted for 8 hours and then cool to room temperature, filtering the inorganic sediments and washing the sediments with alcohol, then the filtrate and wash solution are concentrated under reduced pressure, the residue is dissolved in chloroform (150ml), then extracted successively with 10% sodium hydroxide solution (50ml) and water(50ml x3) to remove the impurity, the chloroform lay is dehydrated with anhydrous sodium sulfate, concentrated under reduced pressure, and the residue is separated by chromatography on an alkaline bauxite hose column , and washed with chloroform to obtain 2,9-diisopropyloxy-1,10-dimethoxy-N-methylaporphine (1.54g, yield is 75%):
   ¹H NMR(200 MHz, CDCl₃) δ 1.40 (6H, d, J=6.1 Hz, 2x CH₃), 1.43 (6H, d, J=6.2 Hz, 2x CH₃), 2.52 (3H, s, NCH₃), 3.64 (3H, s, 1-OCH₃), 3.85 (3H, s, 10-OCH₃), 4.54 (1H, m) and 4.59 (1H, m) (2x OCH), 6.56 (1H, s, H-3), 6.76 (1H, s, H-8), 8.06 (1H, s, H-11). 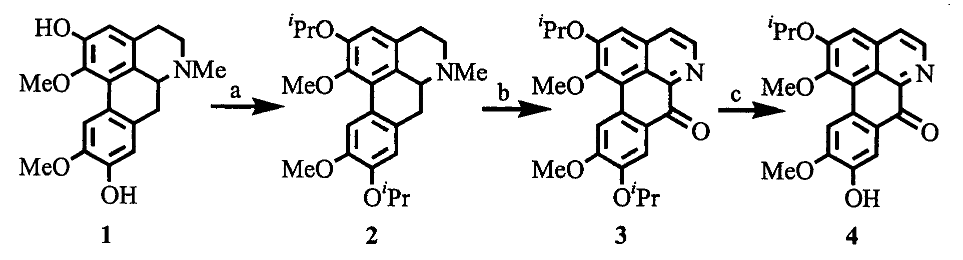
**2. Preparation of 2,9-diisopropyloxy-1,10-dimethoxy-7-oxoaporphine (3),** the formula VII compound, wherein R1=R6=OMe, R2=R5=OiPr, R3=R4=R7=H.
   Lead tetraacetate (95%, 483mg, 1.09mmol) is added into the acetic acid solution (5ml) containing compound (2) (137mg, 330µM), and the reaction is stirred for 12 hours. To the reaction solution, water(150ml) is added, followed by successive extraction with chloroform (50ml × 4), then the chloroform layer is washed successively with saturated sodium bicarbonate aqueous solution(50ml), 10% sodium hyposulfite aqueous solution(50ml) and water(50ml×2), dehydrated with anhydrous sodium sulfate, concentrated with under reduced pressure, and the residue is separated by chromatography on a silica gel hose column, and washed with chloroform to gain 2,9-diisopropyloxy-1,10-dimethoxy-7-oxoaporphine(3) (68mg, 50% yield): m.p. 82-840°C;
   IR(KBr) γₘₐₓ 2976, 2933, 1654, 1590, 1563, 1508, 1459, 1431, 1416, 1359, 1296, 1275, 1241, 1216, 1138, 1110, 1057, 1009, 9956, 925, 887, 864, 782 cm⁻¹;
   ¹H NMR(200 MHz, CDCl₃) δ 1.44 (6H, d, J=6.1 Hz, 2x CH₃), 1.53 (6H, d, J=6.1 Hz, 2x CH₃), 4.01 (3H, s, 1-OCH₃), 3.85 (3H, s, 10-OCH₃), 4.87 (2H, m) (2x OCH), 7.79 (1H, d, J=5.4 Hz, H-4), 7.96 (1H, s, H-8), 8.75 (1H, s, H-11), 8.86 (1H, d, J=5.4 Hz, H-5);
   ¹³C NMR(50 MHz, CDCl₃) δ 21.7 (2C, q), 21.9 (2C, q), 56.0 (q), 60.4 (q), 70.9 (d), 71.2 (d), 107.2 (d), 110.6 (d), 112.2 (d), 120.2 (s), 121.3 (s), 123.2 (d), 126.7 (s), 128.8 (s), 135.4 (s), 14.5 9d), 145.3 (s), 147.8 (s), 151.5 (s), 154.6 (s), 154.8 (s), 181.3 (s);
   ESI MS (positive):M+H⁺ 408.
**3: Preparation of 9-hydroxy-2-isopropyloxy-1,10-dimethoxy-7-oxoaporphine(4),** the formula II compound, wherein R1=R6=OMe, R2=R5=OⁱPr, R3=R4=R7=H.
   Acetic acid-sulfate solution (96:4, 5ml) containing compound (3) is heated under reflux for 1 hour in nitrogen atmosphere. The reaction is cooled to room temperature, then concentrated under reduced pressure, neutralized with ammonia water, extracted with chloroform (100ml x2).The chloroform layer is washed with water(50ml × 2), dehydrated with anhydrous sodium sulfate, concentrated under reduced pressure, and the residue is separated by chromatography on a silica gel hose column, followed by wash with chloroform-methanol (99 : 3) to obtain a brown 9-hydroxy-2-isopropyloxy-1,10-dimethoxy-7-oxoaporphine(4) (10ml, yield is 22%): m.p. 240-242°C;
   IR(KBr) γₘₐₓ3422, 3008, 2977, 2932, 1728, 1651, 1593, 1513, 1461, 1417, 1380, 1351, 1280, 1247, 1213, 1149, 1116, 1059, 1013, 932, 894, 866, 822 cm⁻¹;
   ¹H NMR(200 MHz, CD₃OD) δ 1.51 (6H, d, J=6.0 Hz, 2x CH₃), 4.01 (3H, s, OCH₃), 4.02 (3H, s, OCH₃), 4.87 (1H, m, OCH), 7.25 (1H, s, H-3), 7.74 (1H, s, H-8), 7.83 (1H, d, J=4.8 Hz, H-4), 8.63 (1H, d, J=4.8 Hz, H-5), 8.68 (1H, s, H-11);
   ESI MS (positive):M+H⁺ 366.

### Example 2

### Preparation of 1,10-Dimethoxy-7-oxoaporphine(7), the formula VII compound[, wherein]R1=R6=Me, R2=R3=R4=R5=R7=H.

**1. Preparation of 1,10-dimethoxy-N-methylaporphine(6),** the formula I compound, whereinRl=R7=OMe, R2=R3=R5=R6=R8=H, R4=Me.
   Boldine ((1), 10.0g, 30.58mmol), ethyl cyanide (350ml), anhydrous potassium carbonate (8.4g, 61mmol) and 5-chloro-1-phenyltetrazole (TzCl, 12.14g, 33.64mmol) are placed in a round bottom flask in turn, the mixture is heated under reflux for 24 hour. The reaction is cooled, followed by filtration to remove the inorganic salts, and the sediment is washed with ethyl cyanide . The filtrate and wash solution are concentrated under reduced pressure, and the residue is dissolved in chloroform (400ml), then extracted with water to remove the impurity, the chloroform layer is dehydrated with anhydrous sodium sulfate, concentrated [under reduced pressure, and the residue is separated by chromatography on a silica gel(500g) hose column, then washed with chloroform-methanol(99:1) to obtain 2,9-O-bis(1-phenyltetrazol-5-yl)-1,10-dimethoxy-N-methylaporphine (5) (18g,yield is 96%):
   ¹H NMR(400 MHz, CDCl₃) δ 2.55 (3H, s, NCH₃), 3.46 (3H, s, 1-OCH₃), 3.76 (3H, s, 10-OCH₃), 7.19 (1H, s, H-3), 7.31 (1H, s, H-8), 7.42-7.60 (6H, m) and 7.83-7.87 (4H, m) (C₆H₅×2), 8.04 (1H, s, H-11).

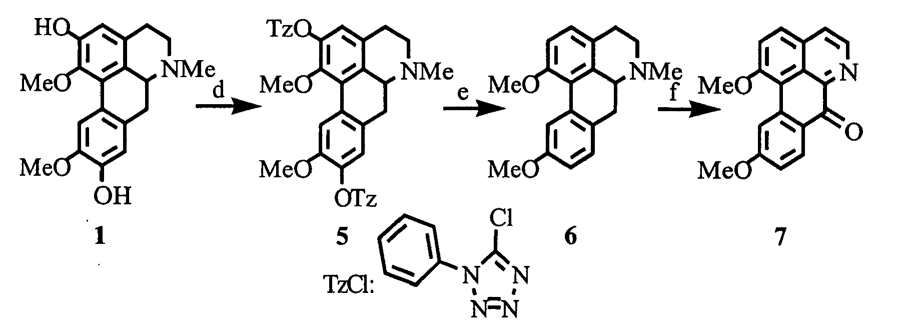

   10% palladium carbon(1g) is added into the acetic acid solution (55ml) containing compound(5) (8g), and then the reaction proceeds for 3 days at 50 °C and 120psi. The reaction is cooled, and palladium carbon is filtered and removed by diatomaceous earth, the filter-off is washed with chloroform, then the filtrate and wash solution are concentrated under reduced pressure, and the residue is dissolved in chloroform (200ml), extracted with 10% sodium hydroxide aqueous solution (50ml × 2) and water(100 × 2) to remove impurities, the chloroform layer is then dehydrated with anhydrous sodium sulfate, concentrated under reduced pressure, and the residue is separated by chromatography on a silica gel hose column, and washed with chloroform-methanol (98:2) to obtain 1,10-dimethoxy-N-methylaporphine (6) (4.12g, yield is 90%):
   ¹H NMR(200 MHz, CDCl₃) δ 2.55 (3H, s, NCH₃), 3.82 (3H, s, 1-OCH₃), 3.85 (3H, s, 10-OCH₃), 6.76 (1H, dd, J=2.7, 8.3 Hz, H-9), 6.87 (1H, d, J=8.5 Hz, H-2), 7.04 (1H, d, J=8.5 Hz, H-3), 7.16 (1H, d, J=8.3 Hz, H-8), 7.89 (1H, d, J=2.7 Hz, H-11);
      ¹³C NMR(50 MHz, CDCl₃) δ 28.3 (t), 33.6 (t), 43.7 (q), 53.1 (t), 55.3 (q), 55.7 (q), 63.1 (d), 110.9 (d), 112.1 (d), 114.7 (d), 121.9 (s), 125.2 (s), 128.1 (d), 128.3 (s), 128.6 (d), 133.0 (s), 136.2 (s), 155.0 (s), 158.1 (s).
**2. Preparation of 1,10-Dimethoxy-7-oxoaporphine(7),** the formula VII compound, wherein R1=R6=Me, R2=R3=R4=R5=R7=H.
   Thallium triacetate (816mg, 2mmol) is added into the acetic acid solution (10ml) containing compound (6) (48mg, 0.5mmol). The reaction proceeds at 70 °C for 1 hour with stirring. Water(150ml) is added into the reaction solution, and then extracted successively with chloroform (50ml×4), the chloroform layer is washed successively with saturated sodium bicarbonate aqueous solution (50ml), 10% sodium hyposulfite aqueous solution (50ml) and water(50ml×2), followed by dehydration with anhydrous sodium sulfate and concentration under reduced pressure. The residue is separated by chromatography on a silica gel hose column, and washed with chloroform-methanol (99:1) to obtain 1,10-dimethoxy-7-oxoaporphine (7) (100mg, yield is 69%): m.p. 162-164°C;
   IR(KBr) γₘₐₓ 2934, 2839, 1644, 1617, 1584, 1540, 1484, 1455, 1406, 1373, 1351,1325, 1255, 1169, 1119, 1048, 1025, 985, 859, 810 cm⁻¹;
   ¹H NMR(400 MHz, CDCl₃-CD₃OD, δ CHCl₃ 7.24) δ 3.79 (3H, s, 10-OCH₃), 4.04 (3H, s, 1-OCH₃), 6.88 (1H, dd, J=2.4, 8.8 Hz, H-9), 7.47 (1H, d, J=9.2 Hz, H-2), 7.73 (1H, d, J=4.7 Hz, H-4), 7.77 (1H, d, J=9.2 Hz, H-3), 8.29 (1H, d, J=8.8 Hz, H-8), 8.35 (1H, d, J=2.4 Hz, H-11), 8.64 (1H, d, J=4.7 Hz, H-5);
   ¹³C NMR(100 MHz, CDCl₃-CD₃OD, δ CDCl₃ 77.0) δ 55.2 (q), 56.3 (q), 112.3 (s), 113.5 (d), 113.8 (d), 119.7(d), 124.9 (d), 125.0 (s), 125.9 (s), 130.7 (d), 130.8 (d), 132.3 (s), 136.6 (s), 141.8 (d), 144.6 (s), 159.0 (s), 164.2 (s), 180.7 (s);
   ESI MS (positive): [M+Na]⁺ 314.

The invention provides an aporphine and oxoaporphine compound for preventing or treating ischemic disease via the mechanism of maintaining or improving the endothelial nitric oxide synthase (eNOS), and said compound can thereby prevent or treat all the ischemic diseases via the eNOS maintaining or improving action; there are no side effects to the patients during treatment of the ischemic disease with the compound, said side effects such as memory loss, body temperature decreasing etc., and thus its effect may be excellent; compared with the conventional methods in the art which can open up blood vessel by lysising the infarcted thrombus, the present invention have a better therapeutic efficacy via loosening and dilatating the blood vessel.

Although the foregoing invention has been described in some detail by way of perfered embodiments, it will be obvious to those skilled in the art that the certain modifications and changes may be practiced within the scope of the invention.

## Claims

1. An aporphine and oxoaporphine compound having the following structure I: 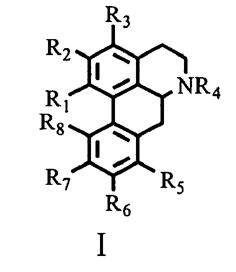 wherein R1, R2, R6 and R7 are selected from H, OH, O-acyl, OMe, OEt, OⁿPr, or OⁱPr, R3 and R5 are selected from H, OH, O-acyl, OMe, F, Cl, Br, NH₂, NO₂ or CN, R4 is selected from Allyl or CₙH2ₙ₊₁, n≥0, R8 is selected from H, OH or OMe.

2. An aporphine and oxoaporphine compound having the following structure II: 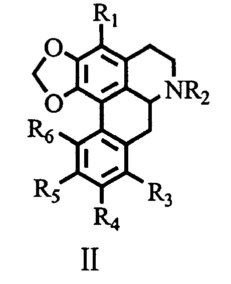 wherein R1 and R3 are selected from H, OH, O-acyl, OMe, F, Cl, Br, NH₂, NO₂ or CN, R2 is selected from allyl or CₙH2ₙ₊₁, n≥0, R7 is selected from H, OH or OMe, R4 and R5 are selected from H, OH, O-acyl, OMe, OEt, OⁿPr or OⁱPr, R6 is selected from H, OH, O-acyl or OMe.

3. An aporphine and oxoaporphine compound having the following structure IIII: 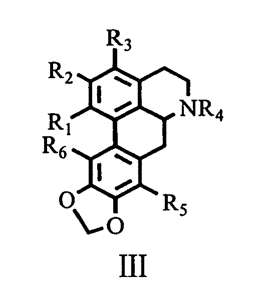 wherein R1 and R2 are selected from H, OH, O-acyl, OMe, OEt, OⁿPr or OⁱPr, R3 and R5 are selected from H, OH, O-acyl, OMe, F, Cl, Br, NH₂, NO₂ or CN, R4 is selected from Allyl or CₙH2ₙ₊₁, n≥0, R6 is selected from H, OH, O-acyl or OMe.

4. An aporphine and oxoaporphine compound having the following structure IV: 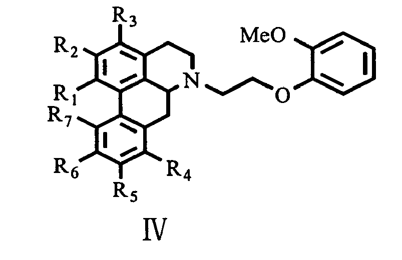 wherein R1, R2, R5 and R6 are selected from H, OH, O-acyl, OMe, OEt, OⁿPr or OⁱPr, R3 and R4 are selected from H, OH, O-acyl, OMe, F, Cl, Br, NH₂, NO₂ or CN, R7 is selected from H, OH, O-acyl or OMe.

5. An aporphine and oxoaporphine compound having the following structure V: 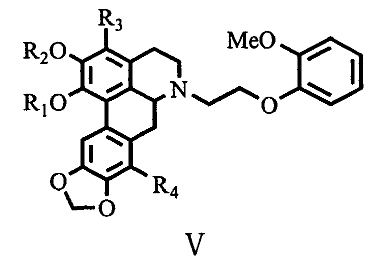 wherein R1 and R2 are selected from H, acyl, Me, Et, OⁿPr or OⁱPr, R3 and R4 are selected from H, OH, O-acyl, OMe, F, Cl, Br, NH₂, NO₂ or CN.

6. An aporphine and oxoaporphine compound having the following structure VI: 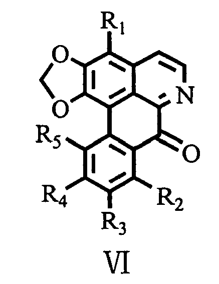 wherein R1 and R2 are selected from H, OH, O-acyl, OMe, F, Cl, Br, NO₂ or CN, R3 and R4 are selected from H, OH, O-acyl, OMe, OEt, OⁿPr or OⁱPr, R5 is selected from H, OH, O-acyl or OMe.

7. An aporphine and oxoaporphine compound having the following structure VII: 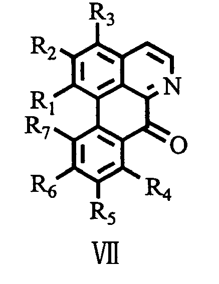 wherein R1, R2, R5 and R6 are selected from H, OH, O-acyl, OMe, OEt, OⁿPr or OⁱPr, R3 and R4 are selected from H, OH, O-acyl, OMe, F, Cl, Br, NO₂ or CN, R7 is selected from H, OH, O-acyl or OMe.

8. An aporphine and oxoaporphine compound having the following structure VIII: 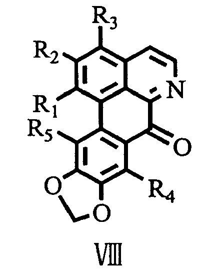 wherein R1 and R2 are selected from H, OH, O-acyl, OMe, OEt, OⁿPr or OⁱPr, R3, and R4 are selected from H, OH, O-acyl, OMe, F, Cl, Br, NO₂ or CN, R5 is selected from H, OH, O-acyl or OMe.

9. The use of an aporphine and oxoaporphine compound as defined in any of claims 1-8 in manufacture of a medicament for proventing or treating ischemic disease.

10. The use of an aporphine and oxoaporphine compound as defined in any of claims 1-8 in manufacture of a medicament for treating ischemic disease in human being or mammal.

11. The use of according to claim 10, wherein the ischemic disease includes ischemic cerebral apoplexy, ischemic cerebral thrombosis, ischemic cerebral embolism, hypoxic ischemic encephlopathy, ischemic cardiac disease and ischemic enteropathy.

12. The use of an aporphine and oxoaporphine compound as defined in any of claims 1-8 in manufacture of a medicament for treating ischemic disease in human being or mammal.

13. The use of according to claim 12, wherein the ischemic disease includes ischemic cerebral apoplexy, ischemic cerebral thrombosis, ischemic cerebral embolism, hypoxic ischemic encephlopathy, ischemic cardiac disease and ischemic enteropathy.

14. A pharmaceutical composition for the prophylaxis or treatment of ischemic disease comprising an effective amount of a compound represented by structure I and a pharmaceutically acceptable carrier or excipient.

15. A pharmaceutical composition for the prophylaxis or treatment of ischemic disease comprising an effective amount of a compound represented by structure II and a pharmaceutically acceptable carrier or excipient.

16. A pharmaceutical composition for the prophylaxis or treatment of ischemic disease I comprising an effective amount of a compound represented by structure III and a pharmaceutically acceptable carrier or excipient.

17. A pharmaceutical composition for the prophylaxis or treatment of ischemic disease comprising an effective amount of a compound represented by structure IV and a pharmaceutically acceptable carrier or excipient.

18. A pharmaceutical composition for the prophylaxis or treatment of ischemic disease comprising an effective amount of a compound represented by structure V and a pharmaceutically acceptable carrier or excipient.

19. A pharmaceutical composition for the prophylaxis or treatment of ischemic disease comprising an effective amount of a compound represented by structure VI and a pharmaceutically acceptable carrier or excipient.

20. A pharmaceutical composition for the prophylaxis or treatment of ischemic disease comprising an effective amount of a compound represented by structure VII and a pharmaceutically acceptable carrier or excipient.

21. A pharmaceutical composition for the prophylaxis or treatment of ischemic disease comprising an effective amount of a compound represented by structure VIII and a pharmaceutically acceptable carrier or excipient.
